# EUROPÄISCHE PATENTSCHRIFT

(11) **EP 3 092 211 B1**
(45) Veröffentlichungstag und Bekanntmachung des Hinweises auf die Patenterteilung: **14.06.2017**
(21) Anmeldenummer: 15710713.7
(22) Anmeldetag: 09.03.2015
(51) Int. Cl.: C07C 1/24, C07C 11/06

(54) **VERFAHREN ZUR HERSTELLUNG VON PROPEN AUS PROPANOL**
PROCESS FOR PREPARING PROPENE FROM PROPANOL
PROCÉDÉ DE FABRICATION DE PROPÈNE À PARTIR DE PROPANOL

(30) Priorität: 10.03.2014 DE 102014003060
(43) Veröffentlichungstag der Anmeldung: 16.11.2016
(73) Patentinhaber: Fraunhofer-Gesellschaft zur Förderung der angewandten Forschung e.V., 80686 München (DE)
(72) Erfinder: FORSTNER, Jochen, 76532 Baden-Baden (DE); UNSER, Steffen, 76287 Rheinstetten (DE); BÖRINGER, Sarah, 76776 Neuburg (DE)
(86) Internationale Anmeldenummer: PCT/EP2015/000521
(87) Internationale Veröffentlichungsnummer: WO 2015/135641

(56) Entgegenhaltungen:
- CN-A- 101 863 731
- DE-A1-102012 200 996
- MASARU WATANABE ET AL: "Conversions of some small organic compounds with metal oxides in supercritical water at 673 KPresented at The First International Conference on Green & Sustainable Chemistry, Tokyo, Japan, March 13?15, 2003.", GREEN CHEMISTRY, Bd. 5, Nr. 5, 1. Januar 2003 (2003-01-01), Seite 539, XP055056339, ISSN: 1463-9262, DOI: 10.1039/b304686a

## Beschreibung

Die Erfindung betrifft ein Verfahren zur Herstellung von Propen mittels Dehydratisierung von 1-Propanol und/oder 2-Propanol an heterogenen Dehydratisierungskatalysatoren.

Propen stellt nach Ethen den wichtigsten organischen Grundstoff für die chemische Industrie dar, wobei Propen beispielsweise verbreitet als Ausgangs- bzw. Zwischenprodukt für die Herstellung von Polypropylen, Acrylnitril, Propenoxid, Phenol, Oxoalkoholen und dergleichen Verwendung findet. Die großtechnische Gewinnung von Propen erfolgt gegenwärtig fast ausschließlich durch Aufspaltung, dem sogenannten Cracken, von fossilen Brennstoffen, beispielsweise durch thermisches Cracken, katalytisches Cracken oder Steamcracken. Nachteilig ist einerseits die begrenzte Verfügbarkeit der als Rohstoff eingesetzten fossilen Brennstoffe, andererseits fällt das Propen auf diese Weise als Nebenprodukt bei der Gewinnung von Benzinen und Ethen an, so dass ein steigender Bedarf an Propen nur einhergehend mit einer steigenden Produktion von Benzinen und Ethen gedeckt werden kann.

Um dem zu begegnen, sind in jüngerer Zeit petrochemische Verfahren zur Herstellung von Propen entwickelt worden, wie beispielsweise die Metathese oder die sogenannten "Methanol-to-Olefins"-Verfahren. Im Falle der Herstellung von Propen mittels der erstgenannten Metathese erfolgt die Umsetzung von Buten und Ethen zu Propen an WO₃/SiO₂-Katalysatoren, wobei auf diese Weise insbesondere die Möglichkeit besteht, das Propen praktisch gänzlich aus nachwachsenden Rohstoffen zu erzeugen. Letzteres kann durch Fermentation von natürlichen Zuckern zu Ethanol und anschließender Dehydratisierung des Ethanols zu Ethen geschehen, wobei der notwendige Anteil an Buten durch Dimerisierung des Ethens erzeugt wird. Als problematisch hat sich allerdings insbesondere die relativ geringe Selektivität der Metathesereaktion für Propen erwiesen, wobei neben dem Propen in großem Umfang auch Alkane und aromatische Verbindungen erzeugt werden, was eine Gewinnung des Propens in zufriedenstellender Reinheit sehr aufwändig und teuer macht.

Darüber hinaus sind zur Herstellung von Propen gattungsgemäße Dehydratisierungsverfahren bekannt, bei welchen die entsprechenden Alkohole, nämlich 1-Propanol oder 2-Propanol, unter Abspaltung von Wasser katalytisch zu Propen umgesetzt werden. So beschreibt die US 2009/0281362 A1 ein katalytisches Dehydratisierungsverfahren von 1-Propanol zu Propen in der Gasphase bei Temperaturen von 160°C bis 270°C und Drücken von 10 bar bis 45 bar an verschiedenen Festkörperkatalysatoren. Ein Einsatz von 2- bzw. iso-Propanol als Edukt, wie es vornehmlich bei der Gewinnung aus nachwachsenden Rohstoffen mittels biotechnologischer Verfahren, wie der Fermentation von Zuckern, Lignocellulose und dergleichen anfällt, ist offenbar nicht möglich.

Darüber hinaus offenbart die WO 2011/161045 A1 ein Verfahren zur Dehydratisierung von unter anderem Propanol in der Gasphase bei etwa 550°C und 2 bar an einem phosphormodifizierten Zeolith-Katalysator (P-ZSM-5) mit einem Silicium-/ Aluminiumoxidverhältnis von größer 10.

Ferner beschreibt der Aufsatz "Conversions of some small organic compounds with metal oxides in supercritical water at 673°K, presented at the First International Conference on Green & Sustainable Chemistry, Tokyo, Japan, March 13-15, 2003" von Masaru Watanabe et al. (Green Chemistry 5, Nr. 5, 01.01. 2013, Seite 539, XPO55056339) die überkritische Dehydratisierung von unter anderem Propanol zu Propen an heterogenen Katalysatoren auf der Basis von Kupfer-, Molybdän-, Titan- und Zinkoxiden.

Schließlich ist der DE 10 2012 200 996 A1 ein Verfahren zur katalytischen Dehydratisierung von Ethanol zu Ethen zu entnehmen, welches unter anderem auch als geeignet für die Dehydratisierung von Propanolen zu Propen beschrieben wird. Indes hat sich jedoch gezeigt, dass dieses bekannte Verfahren hierfür nur sehr bedingt geeignet ist, wobei insbesondere eine zunehmende Zersetzung der Katalysatoren in dem Reaktor beobachtet wurde und sich die Abtrennung des Propens aus dem erhaltenen Produkt als praktisch unmöglich erwiesen hat. Letzteres hängt damit zusammen, dass der überkritische Produktstrom aus dem Reaktor zwar auf eine Temperatur unterhalb 50°C abgekühlt wird, aber der Reaktordruck von wenigstens 220 bar aufrechterhalten werden soll, so dass sowohl Ethen als auch insbesondere Propen nach wie vor im überkritischen bzw. flüssigen Zustand vorliegt, in welchem es in (unterkritischem) Wasser gut mischbar ist bzw. zumindest als weitere flüssige Phase aus dem Phasenseparator ausgetragen wird, so dass dem Phasenseparator stets eine Mischung aus Propen und Wasser entnommen wird. Zudem hat sich gezeigt, dass es bei der Entnahme einer solchen Mischung aus dem Phasenseparator aufgrund der Entspannung dieser Mischung einerseits zu einem Ausgasen des gewünschten Produktes (Propen) aus dem Wasser kommt, andererseits sich das Entnahmeventil stets aufgrund Gefrierens der entnommenen Mischung zusetzt, wenn nicht nur die Kompressionswärme des Propens, sondern auch dessen Verdampfungswärme freiwird.

Der Erfindung liegt die Aufgabe zugrunde, unter zumindest weitestgehender Vermeidung der vorgenannten Nachteile ein gattungsgemäßes Verfahren zur Herstellung von Propen mittels Dehydratisierung von 1-Propanol und/oder 2-Propanol an heterogenen Dehydratisierungskatalysatoren vorzuschlagen, welches eine Schädigung des Katalysators auch bei langer Standzeit weitestgehend verhindert, eine sehr hohe Selektivität der Umsetzung sowohl von 1-Propanol als auch insbesondere von 2-Propanol (iso-Propanol) zu Propen aufweist und dabei auch den Einsatz verunreinigter Edukte ermöglicht, wie sie beispielsweise durch biologischen Abbau natürlicher Rohstoffe erhalten werden, eine einfache Abtrennung des Propens aus dem Produkt ermöglicht sowie möglichst energiesparend durchführbar ist.

Erfindungsgemäß wird diese Aufgabe mit einem Verfahren der eingangs genannten Art gelöst, welches die folgenden Schritte umfasst:
(a) Bereitstellen eines 1-Propanol und/oder 2-Propanol ententhaltenden Eduktes und Erwärmen des Eduktes auf wenigstens 374°C sowie Beaufschlagen des Eduktes mit einem Druck von wenigstens 221 bar;
(b) Überführen des Eduktes in wenigstens einen mit wenigstens einem heterogenen Dehydratisierungskatalysator befüllten Reaktor und Inkontaktbringen des Eduktes mit dem Dehydratisierungskatalysator unter Aufrechterhaltung einer Temperatur von wenigstens 374°C sowie einem Druck von wenigstens 221 bar, so dass ein Propen enthaltendes Produkt erhalten wird;
(c) Entspannen des Produktes auf höchstens 47 bar sowie Abkühlen des Produktes auf höchstens 90°C stromab des Reaktors und Überführen des Produktes in einen Phasenseparator; und
(d) Entnehmen des Propens aus der Gasphase des Phasenseparators und Entnehmen zumindest des bei der Dehydratisierungsreaktion gebildeten Wassers aus der Flüssigphase des Phasenseparators unter Aufrechterhaltung einer Temperatur von höchstens 90°C sowie einem Druck von höchstens 47 bar in dem Phasenseparator.

Das erfindungsgemäße Verfahren stellt aufgrund des in dem Reaktor eingestellten, stets überkritischen Zustandes von Wasser, wie es bei der Reaktion von Propanolen zu Propen abgespalten wird oder wie es in dem Edukt als Lösungsmittel eingesetzt werden kann (siehe hierzu weiter unten), zunächst sicher, dass der heterogene Katalysator nicht mit Wasserdampf bzw. insbesondere flüssigem Wasser in Berührung kommt, so dass eine Beeinträchtigung des Katalysators auch bei langer Standzeit zuverlässig vermieden wird. Das Edukt wird daher erfindungsgemäß bereits stromauf des Reaktors auf eine Temperatur von wenigstens etwa 374°C erwärmt und auf einen Druck von wenigstens etwa 221 bar komprimiert, wobei der Reaktor zweckmäßigerweise beheizt wird, um diese Temperatur während der endothermen Dehydratisierungsreaktion zumindest aufrechtzuerhalten. Hiermit geht auch eine Energieeinsparung im Vergleich mit einer Gasphasendehydratisierung gemäß dem Stand der Technik einher, da das mehr oder minder reine oder insbesondere in gelöster Form eingesetzte Propanol nicht verdampft werden muss, sondern die Reaktion in komprimierter Phase stattfindet. Letzteres resultiert überdies in einer außerordentlich hohen Raum-ZeitAusbeute.

Die Selektivität der Umsetzung sowohl von 1-Propanol als auch von 2-Propanol beträgt bei diesen Bedingungen weit über 90% und zumeist deutlich über 95%, so dass sowohl 1-Propanol als auch insbesondere das bei Fermentationsprozessen aus biologischen Abbauvorgängen von nachwachsenden Rohstoffen anfallende 2- bzw. iso-Propanol als Produkt eingesetzt werden kann. Dabei ist die Dehydratisierungsreaktion höchst tolerant gegenüber etwaigen Verunreinigungen des Produktes, wie beispielsweise gegenüber anderen Alkoholen oder auch Ethern, Aldehyden, Ketonen oder dergleichen, so dass insbesondere im Falle des Einsatzes von Fermentationslösungen eine aufwändige Aufbereitung bzw. Reinigung entbehrlich ist. Darüber hinaus können auch zumindest einige solcher Verunreinigungen bei den eingestellten Reaktionsparametern zu Propen umgesetzt werden, wobei z.B. festgestellt worden ist, dass in dem Produkt enthaltenes Aceton (Dimethylketon), wie es beispielsweise bei der sogenannten ABE-Fermentation (Aceton/Butanol/Ethanol-Fermentation) aus Zuckern oder Lignocellulose anfällt und auch nach einer Reduktion des Acetons zu 2-Propanol üblicherweise zumindest in Spuren noch enthalten ist, zu Propen mit umgesetzt wird, wobei vermutet wird, dass das Aceton durch zumindest intermediär in dem Reaktor gebildeten Wasserstoff zu 2-Propanol hydriert wird, welcher dann unmittelbar zu Propen umgesetzt wird. Desgleichen beträgt der Umsatz an 2-Propanol bei diesen Reaktionsbedingungen weit über 90% und beträgt auch der Umsatz von 1-Propanol jedenfalls deutlich mehr als 50%.

Ferner stellt das erfindungsgemäße Verfahren insbesondere eine sehr einfache Abtrennung des erzeugten Propens aus der Gasphase des stromab des Reaktors angeordneten Phasenseparators sicher, indem der Phasenseparator stets auf einem Druck von höchstens etwa 47 bar, insbesondere unterhalb 47 bar, sowie einer Temperatur von höchstens etwa 90°C gehalten wird, so dass das Propen gasförmig vorliegt, während das bei der Reaktion gebildete oder auch in dem Produkt als Lösungsmittel enthaltene Wasser in flüssiger Phase vorliegt und etwaige nicht umgesetzte Reste des eingesetzten Propanols sowie gegebenenfalls mögliche flüssige, bei der Dehydratisierungsreaktion gebildete Nebenprodukte, wie z.B. geringe Anteile an Aceton oder 1-Propanol im Falle des Einsatzes von 2-Propanol, hierin gelöst sind. Erfindungswesentlich ist hierbei, dass das stromab des Reaktors und vorzugsweise stromauf des Phasenseparators expandierte Produkt nicht nur in einen für Propen unterkritischen Zustand überführt wird, sondern dass die Parameter Druck und Temperatur dabei derart eingestellt werden, dass das Propen in der Gasphase vorliegt, nachdem sich gezeigt hat, dass eine (unterkritische) Abtrennung von flüssigem Propen aus (flüssigem) Wasser mit ähnlichen Problemen behaftet ist wie die überkritische Abtrennung von Ethen/Propen aus Wasser gemäß der oben zitierten DE 10 2012 300 996 A1. Damit das Propen in dem Phasenseparator im gasförmigen Zustand vorliegt, muss der Druck, auf welchen das Propen entspannt wird, folglich an die Temperatur angepasst werden, so dass sich das Propen in seinem Phasendiagramm (p,T-Diagramm) in der Gasphase befindet. Das Phasendiagramm von Propen steht dem Fachmann in der einschlägigen Literatur, in Produktdatenblättern von kommerziellen Anbietern von Propen oder im Internet (z.B. unter http://encyclopedia.airliquide.com/ images encyclopedie/VaporPressureGraph/Propylene Vapor Pressure.GIF) vielfach zur Verfügung. Das somit als Produktgas anfallende Propen kann im Übrigen vorteilhafterweise noch unter einem Druck (knapp) unterhalb etwa 47 bar stehen, so dass eine energieintensive Kompression anlässlich der Abfüllung oder Weiterverarbeitung entbehrlich wird.

Darüber hinaus bietet sich das erfindungsgemäße Verfahren vorzugsweise für eine kontinuierliche Durchführung an, wobei es insbesondere die folgenden Schritte umfassen kann:
(a) Bereitstellen eines 1-Propanol und/oder 2-Propanol enthaltenden Eduktstromes und Erwärmen des Eduktstromes auf wenigstens 374°C sowie Beaufschlagen des Eduktstromes mit einem Druck von wenigstens 221 bar;
(b) Überführen des Eduktstromes in wenigstens einen mit wenigstens einem heterogenen Dehydratisierungskatalysator befüllten Reaktor, insbesondere in Form eines Rohrreaktors, eines Rohrbündelreaktors oder einer Kaskade der vorgenannten Reaktoren, und Inkontaktbringen des Eduktstromes mit dem Dehydratisierungskatalysator unter Aufrechterhaltung einer Temperatur von wenigstens 374°C sowie einem Druck von wenigstens 221 bar, so dass ein Propen enthaltender Produktstrom erhalten wird;
(c) Entspannen des Produktstromes auf höchstens 47 bar sowie Abkühlen des Produktstromes auf höchstens 90°C stromab des Reaktors und Überführen des Produktstromes in einen Phasenseparator; und
(d) Entnehmen eines Propenstromes aus der Gasphase des Phasenseparators und Entnehmen zumindest eines Flüssigkeitsstromes, welcher das bei der Dehydratisierungsreaktion gebildete Wasser enthält, aus der Flüssigphase des Phasenseparators unter Aufrechterhaltung einer Temperatur von höchstens 90°C sowie einem Druck von höchstens 47 bar in dem Phasenseparator.

Wie bereits angedeutet, kann in vorteilhafter Ausgestaltung vorgesehen sein, als Edukt eine wässrige Lösung von 1-Propanol und/oder 2-Propanol, insbesondere in einer Konzentration zwischen etwa 5 und etwa 98 Mass.-%, vorzugsweise in einer Konzentration zwischen etwa 6 und etwa 96 Mass.-%, 1- und/oder 2-Propanol bezogen auf die Gesamtlösung, eingesetzt wird. Dabei bietet das erfindungsgemäße Verfahren insbesondere auch die Möglichkeit des Einsatzes von niederkonzentrierten wässrigen Propanollösungen, so dass insbesondere im Falle des Einsatzes von aus natürlichen Rohstoffen gewonnenen Fermentationslösungen etwaige Aufbereitungen bzw. Aufkonzentrierungen entbehrlich sind, was mit einer entsprechenden Energieersparnis einhergeht.

In bevorzugter Ausführung kann in dem Reaktor eine Temperatur zwischen etwa 374°C und etwa 500°C und/oder ein Druck zwischen etwa 221 bar und etwa 350 bar eingestellt werden, wie es sich hinsichtlich eines hohen Umsatzes und insbesondere einer hohen Selektivität der Dehydratisierungsreaktion zu Propen als günstig erwiesen hat.

Ferner kann in bevorzugter Ausführung das Produkt stromab des Reaktors auf eine Temperatur zwischen etwa 5°C und etwa 90°C, insbesondere zwischen etwa 60°C und etwa 90°C, abgekühlt und/oder auf einen Druck zwischen etwa 1 bar und etwa 46 bar, insbesondere zwischen etwa 30 bar und etwa 46 bar, entspannt werden, wobei die genannten Temperatur- und Druckintervalle zweckmäßig in dem Phasenseparator aufrechterhalten werden, so dass das erzeugte Propen in der gasförmige Phase vorliegt. Insoweit sei auf die Ausführungen weiter oben in Bezug auf die Wechselwirkung des Druckes mit der Temperatur hingewiesen, um das Propen in dem Phasenseparator in der Gasphase von dem flüssigen Wasser abzutrennen.

Gemäß einer vorteilhaften Weiterbildung des erfindungsgemäßen Verfahrene kann vorgesehen sein, dass zumindest ein Teil der aus dem Phasenseparator entnommenen flüssigen Phase auf wenigstens 374°C erwärmt sowie mit einem Druck von wenigstens 221 bar beaufschlagt und in den Reaktor rezirkuliert wird. Auf diese Weise können nicht nur Reste nicht umgesetzten Propanols, sondern auch etwaige Verunreinigungen oder Nebenprodukte, gegenüber welchen das Verfahren, wie oben erwähnt, sehr tolerant ist, praktisch vollständig umgesetzt werden. Der Rezirkulationsstrom kann dabei insbesondere dem Eduktstrom zugesetzt und gemeinsam mit diesem stromauf des Reaktors auf wenigstens etwa 374°C erwärmt sowie auf wenigstens etwa 221 bar komprimiert werden.

Das erfindungsgemäße Verfahren gibt insbesondere auch die Möglichkeit, das dem Phasenseparator entnommene Propen einem Abfüll- oder Weiterverarbeitungsprozess zuzuführen, wobei in diesem Fall der Druck in dem Phasenseparator vorzugsweise möglichst hoch, z.B. zwischen etwa 30 bar und etwa 47 bar, vorzugsweise zwischen etwa 35 bar und etwa 47 bar, insbesondere zwischen etwa 40 bar und etwa 47 bar, eingestellt werden sollte (d.h. das aus dem Reaktor austretende Produkt wird auf einen solchen Druck entspannt), damit für etwaige Weiterverarbeitungsprozesse, wie beispielsweise einer Destillation und/oder einer chemischen Umsetzung zu den gewünschten Folgeprodukten, keine erneute, energieintensive Kompression des gewonnenen Propens stattfinden muss, sondern der "Restdruck" des Propens aus dem Reaktor genutzt werden kann. Entsprechendes gilt selbstverständlich beispielsweise für eine Abfüllung des Propens anlässlich dessen Bevorratung, Transportes etc.

Als heterogener Katalysator kann in vorteilhafter Ausgestaltung wenigstens ein Katalysator auf der Basis von Metalloxiden aus der Gruppe der Metalle aus der vierten Nebengruppe und aus der dritten Hauptgruppe des Periodensystems, insbesondere aus der Gruppe Aluminium, Zirconium und Titan, eingesetzt werden. Als besonders geeignet für das erfindungsgemäße Verfahren haben sich als heterogene Katalysatoren dabei solche aus der Gruppe Aluminiumoxid (Al₂O₃), insbesondere der kubischen Modifikation γ-Aluminiumoxid und/oder θ-Aluminiumoxid, Zirconiumdioxid (ZrO₂), insbesondere in tetragonaler Modifikation, und Titandioxid (TiO₂), insbesondere in tetragonaler holoedrischer Modifikation (Anatas), erwiesen, welche vorzugsweise zumindest als Hauptkomponente heterogener Dehydratisierungskatalysatoren eingesetzt werden können. Die genannten Katalysatoren sorgen für hohe Umsätze sowohl von 1- als auch insbesondere von 2-Propanol bei einer sehr hohen Selektivität für Propen, wobei anlässlich mittels HPLC durchgeführter Analysen eines aus einer wässrigen 2-Propanollösung erhaltenen Produktes lediglich sehr geringe Mengen an Propanolresten sowie Aceton und Propionaldehyd in der flüssigen Phase des Phasenseparators nachgewiesen werden konnten, während etwa die Bildung von Dipropylethern nicht observiert werden konnte. Gaschromatographische Untersuchungen des gasförmigen Produktes (aus der Gasphase des Phasenseparators) ergaben neben Propen sehr geringe Spuren an Wasserstoff, Propan, Ethan, Ethen, Methan, Kohlenmonoxid und Kohlendioxid.

Ferner hat es sich als zweckmäßig erwiesen, wenn wenigstens ein heterogener Katalysator, wie vorzugsweise ein solcher aus der oben genannten Gruppe, mit einer spezifischen Oberfläche zwischen etwa 10 m²/g und etwa 400 m²/g, ermittelt nach BET (Brunauer, Emmett, Teller), eingesetzt wird.

Der Reaktor kann entweder im Wesentlichen vollständig mit einer Schüttung der heterogenen Katalysatormaterialien befüllt werden, so dass der Produktstrom deren Hohlraumvolumen passiert, oder er kann nur teilweise bzw. unvollständig mit dem Dehydratisierungskatalysator befüllt werden, was im Hinblick darauf vorteilhaft sein kann, dass es sich bei der Dehydratisierung von Propanolen zu Propen um eine endotherme Reaktion handelt und auf diese Weise auch lokale und/oder kurzzeitige Abkühlungen der in dem Reaktor befindlichen Reaktionsmischung auf unterhalb etwa 374°C vermieden werden können bzw. eine moderatere Beheizung des Reaktors möglich ist. So kann die Beladung eines kontinuierlich betriebenen Reaktors mit dem heterogenen Katalysator z.B. zwischen etwa 0,001 und etwa 3 g_{Propanol}/g_{Katalysator} · min, insbesondere zwischen etwa 0,005 und etwa 2 g_{Propanol}/g_{Katalysator} · min, betragen. Lediglich exemplarisch sei im Hinblick auf eine vorteilhafte Verweilzeit (ausgedrückt durch das Verhältnis des Massenstromes durch den Reaktor bezogen auf das freie Reaktorvolumen) des Produktstromes in dem Reaktor ergänzt, dass sich z.B. im Falle eines einem kontinuierlichen Rohrreaktor mit einem freien Füllvolumen von etwa 100 ml aufgegebenen Produktstromes von 30 ml/min einer 40 Mass.-%igen wässrigen iso-Propanollösung (entsprechend 27,6 g/min bei 250 bar) Werte zwischen etwa 250 g/l · min und etwa 300 g/l · min als zweckmäßig erwiesen haben.

Wie weiter oben bereits erwähnt, kann das für das erfindungsgemäße Verfahren eingesetzte 1-Propanol und/oder insbesondere 2-Propanol vorzugsweise aus nachwachsenden Rohstoffen, insbesondere mittels Fermentation, z.B. aus Zuckern, Lignocellulose und dergleichen, gewonnen werden, wobei selbstverständlich auch Propanole synthetischen Ursprungs eingesetzt werden können, wie sie insbesondere aus Spalt- bzw. Crackprozessen und diesen nachgeschalteten Prozessen von fossilen Brennstoffen gewonnen werden.

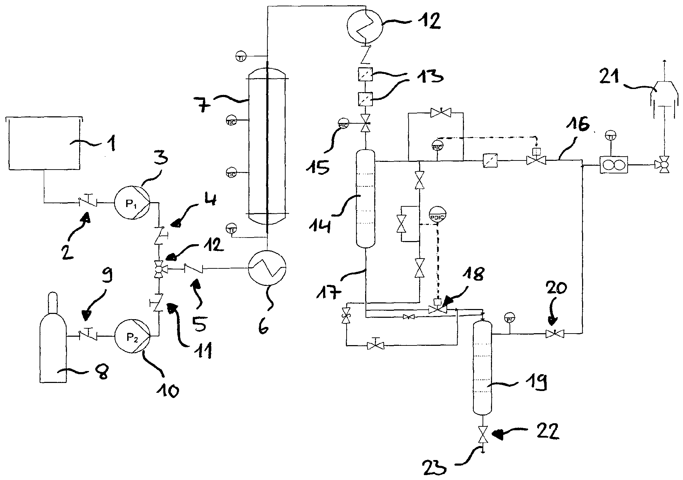

Weitere Merkmale und Vorteile der Erfindung ergeben sich aus der nachfolgenden Beschreibung von Ausführungsbeispielen des Verfahrens zur Herstellung von Propen mittels Dehydratisierung von Propanolen an heterogenen Dehydratisierungskatalysatoren unter Bezugnahme auf die Zeichnung. Dabei zeigt die einzige Figur:
ein schematisches Fließbild einer Ausführungsform einer Vorrichtung zur Durchführung eines solchen Verfahrens.

Die in der Zeichnung schematisch wiedergegebene Ausführungsform einer kontinuierlich arbeitenden Vorrichtung zur Herstellung von Propen umfasst einen Vorlagebehälter 1 zur Aufnahme eine Eduktes, wie einer wässrigen Lösung aus 1- und/oder 2-Propanol. Aus dem Vorlagebehälter 1 mündet eine mit einem Sperrventil 2 versehene Auslassleitung in eine Pumpe 3, welche zum Komprimieren eines dem Vorlagebehälter 1 entnommenen Eduktstromes auf wenigstens etwa 221 bar dient. Stromab der Pumpe 3 schließt sich an diese eine mit einem weiteren Sperrventil 4 versehene Leitung an, welche stromab eines weiteren Sperrventils 5 in einem Wärmetauscher 6 mündet, um den bereits druckbeaufschlagten Eduktstrom auf eine Temperatur von wenigstens etwa 374°C zu erwärmen, so dass sie sich in einem überkritischen Zustand befindet. Der Wärmetauscher 6 ist wiederum mit einem Reaktor 7 verbunden, welcher mit einem geeigneten heterogenen Dehydratisierungskatalysator, beispielsweise γ-Aluminiumoxid, befüllt ist, an welchem das in dem Eduktstrom enthaltene Propanol zu Propen umgesetzt wird. Der Reaktor 7 ist insbesondere beheizt (nicht gezeigt), um ein auch lokales Abkühlen der Reaktionslösung auf unterhalb etwa 374°C zuverlässig zu verhindern.

Darüber hinaus umfasst die Vorrichtung beispielsweise ein Druckgasreservoir 8, welches insbesondere mit einem Inertgas, wie Stickstoff, Kohlendioxid oder dergleichen, befüllt ist, wobei aus dem Druckgasreservoir 8 gleichfalls eine mit einem Sperrventil 9 versehene Auslassleitung in eine weitere Pumpe 10 mündet. Stromab der Pumpe 10 schließt sich an diese eine mit einem weiteren Sperrventil 11 versehene Leitung an, welche über ein Ventil 12, z.B. in Form eines Drei- oder Mehrwegeventils, in die den Eduktstrom führende Leitung mündet, und zwar beispielsweise stromab der Pumpe 3 und stromauf des Wärmetauschers 6. Das Inertgas dient dabei vornehmlich zum Spülen des Reaktors 7 sowie zum Aufbau des erforderlichen Druckes in demselben beim Anfahren der Vorrichtung, bevor die Eduktlösung in den Reaktor 7 geleitet wird.

Stromab des Reaktors 7 gelangt die Produktlösung über eine Abführleitung zunächst in einen weiteren Wärmetauscher 12, wo sie auf eine Temperatur von höchstens etwa 90°C, z.B. auf etwa 80°C oder auf etwa 65°C, abgekühlt wird. Der stromab des Reaktors 7 angeordnete, zur Abkühlung des Produktstromes dienende Wärmetauscher 12 kann aus energieökonomischen Gründen insbesondere mit dem zur Erwärmung des Eduktstromes dienenden Wärmetauscher 6 verbunden sein, um einen geschlossenen Kühl- bzw. Heizmittelkreislauf zu bilden. Wiederum stromab des Wärmetauschers 12 gelangt die Produktlösung schließlich, beispielsweise unter Zwischenschaltung eines oder mehrerer Filter 13, in einen Phasenseparator 14, wobei die letzteren mit dem Reaktor 7 verbindende Leitung mit einem Druckregelventil 15 versehen ist, um den Produktstrom auf einen Druck von unterhalb 47 bar, beispielsweise auf etwa 35 bar (bei 80°C) oder auf etwa 30 bar (bei 65°C), bei welchem sich das erzeugte Propen bei den oben genannten 80°C bzw. 65°C jeweils in der Gasphase befindet, zu entspannen und diesen Druck in dem Phasenseparator aufrechtzuerhalten, während zugleich der Reaktordruck auf einem Wert oberhalb etwa 221 bar gehalten wird.

Dem Phasenseparator 14 ist einerseits aus der Gasphase das erzeugte Propen über eine mit einer Anordnung von Drucksteuerventilen versehene Leitung 16 zu entnehmen; andererseits ist dem Phasenseparator 14 die flüssige, wässrige Phase über eine gleichfalls mit einer Anordnung von Drucksteuerventilen einschließlich eines Füllstandsregelventils 18 versehene Leitung 17 zu entnehmen, welche in einen Auffangbehälter 19 mündet. Der Gasraum des Auffangbehälters 19 steht beispielsweise über eine mit einem Ventil 20 ausgestattete Verbindungsleitung mit der das aus dem Phasenseparator 14 abgezogene Propen enthaltenden Leitung 16 in Verbindung, stromab derselben sich eine Entnahmestelle 21 für das Propen befindet, an welche sich ein oder mehrere Weiterverarbeitungsprozesse anschließen können (nicht zeichnerisch wiedergegeben). Die flüssige Phase des Auffangbehälters 19 kann insbesondere über eine mit einem Sperrventil 22 versehene Rezirkulationsleitung 23 in den Reaktor 7 rezirkuliert werden, wobei die Rezirkulationsleitung 23 beispielsweise in den Vorlagebehälter 1 münden kann, um die rezirkulierte flüssige Phase gemeinsam mit der frischen Eduktlösung - nach Druckbeaufschlagung und Erwärmung derselben - wieder dem Reaktor 7 aufzugeben (nicht zeichnerisch dargestellt).

### Ausführungsbeispiele:

### Beispiel 1:

In der oben beschriebenen Versuchsvorrichtung wurde eine 40 Mass.-%ige 2-Propanollösung umgesetzt. Zu diesem Zweck wurde der Reaktor 7 im Wesentlichen vollständig mit 60,0 g γ-Aluminiumoxid als heterogenem Dehydratisierungskatalysator befüllt. Das verbleibende freie Reaktorvolumen berechnete sich zu etwa 100,3 ml. Als Reaktionsparameter wurden 240 bar Reaktordruck und 394°C Reaktorinnentemperatur eingestellt. Der Volumenstrom der Eduktlösung durch die Pumpe 3 betrug 30 ml/min. Der Druck des Phasenseparators 14 betrug 2 bar. Aus diesen Versuchsparametern resultierte ein Umsatz des 2-Propanols von 94,95%; die Selektivität zu Propen betrug 96,32%.

### Beispiel 2:

Analog Beispiel 1 wurde der Reaktor 7 mit 61,1 g γ-Aluminiumoxid geringerer Oberfläche befüllt. Hieraus resultierte ein freies Reaktorvolumen von etwa 99,5 ml. Als Reaktionsparameter wurden 251 bar Reaktordruck und 398°C Reaktorinnentemperatur eingestellt. Der Volumenstrom durch die Pumpe 3 war wiederum 30 ml/min bei einer 40 Mass.-%igen 2-Propanollösung. Der Druck des Phasenseparators 14 betrug wiederum 2 bar. Hieraus resultierte ein Umsatz des 2-Propanols von 93,31%, der Volumenanteil von Propen in der Gasphase errechnete sich zu 98,23%.

### Beispiel 3:

Analog Beispiel 1 wurde der Reaktor 7 mit 143,4 g Zirconiumdioxid (tetragonal) als heterogenem Dehydratisierungskatalysator befüllt. Hieraus resultierte ein freies Reaktorvolumen von 92,0 ml. Als Reaktionsparameter wurden 240 bar Reaktordruck und 396°C Reaktorinnentemperatur eingestellt. Der Volumenstrom durch die Pumpe 3 war wiederum 30 ml/min bei einer 40 Mass.-%igen 2-Propanollösung. Der Druck des Phasenseparators 14 wurde wiederum auf 2 bar eingestellt. Hieraus resultierte ein Umsatz des 2-Propanols von 96,64%; die Selektivität zu Propen betrug 99,31%.

### Beispiel 4:

Analog Beispiel 1 wurde der Reaktor 7 wurde mit 67,6 g θ-Aluminiumoxid als heterogenem Dehydratisierungskatalysator befüllt. Hieraus resultierte ein freies Reaktorvolumen von 102,8 ml. Als Reaktionsparameter wurden 260 bar Reaktordruck und 406°C Reaktorinnentemperatur eingestellt. Der Volumenstrom durch die Pumpe 3 betrug 65 ml/min; die Konzentration der vorgelegten 2-Propanollösung betrug 19,5 Mass.-%. Der Druck des Phasenseparators wurde wiederum auf 2 bar eingestellt. Hieraus resultiert ein Umsatz des 2-Propanols von 95,4 %; die Selektivität zu Propen betrug 97,3%.

### Beispiel 5:

Analog Beispiel 1 wurde der Reaktor 7 mit 81,9 g Titandioxid in tetragonal holoedrischer Modifikation (Anatas) befüllt. Hieraus resultierte ein freies Reaktorvolumen von 101,2 ml. Als Parameter wurden 250 bar Reaktorruck und 402°C Reaktorinnentemperatur eingestellt. Der Volumenstrom durch die Pumpe 3 war 30 ml/min. Der Druck im Phasenseparator 14 betrug wiederum 2 bar. Hieraus resultiert ein Umsatz des 2-Propanols von 97,13%; die Selektivität zu Propen betrug 94,39%.

### Beispiel 6:

Es wurden dem Reaktor 7 67,6 g θ-Aluminiumoxid als heterogener Dehydratisierungskatalysator aufgegeben. Hieraus ergab sich ein freies Reaktorvolumen von 102,8 ml. Der Reaktor 7 wurde mit einer wässrigen Lösung von 40 Mass.-% 1-Propanol beschickt. Die Reaktionstemperatur im Reaktor 7 betrug 408°C, der Reaktionsdruck 250 bar. Der Volumenstrom der Pumpe 3 betrug wiederum 30 ml/min. Durch diese Betriebsweise ergab sich ein Umsatz des 1-Propanols von 55,29%; die Selektivität zu Propen belief sich auf 92,8%.

### Beispiel 7:

Analog Beispiel 1 wurde der Reaktor 7 mit einer wässrigen Eduktlösung von 96,0 Mass.-% 2-Propanol durchströmt. Das freie Reaktorvolumen nach Befüllung mit 66,5 g θ-Aluminiumoxid als heterogenem Dehydratisierungskatalysator betrug 97,4 ml. Hieraus ergab sich bei einem Reaktordruck von 240 bar, einer Reaktortemperatur von 400°C und einem Volumenstrom von wiederum 30 ml/min ein Umsatz des 2-Propanols von 63,53%. Die Selektivität bezüglich Propen errechnete sich zu 97,83%.

### Beispiel 8:

Analog Beispiel 1 wurde der Reaktor 7 mit einer Eduktlösung von 10,1 Mass.-% 2-Propanol durchströmt. Das freie Reaktorvolumen nach Befüllung mit 60,5 g γ-Aluminiumoxid als heterogenem Dehydratisierungskatalysator betrug 102,4 ml. Hieraus ergab sich bei einem Reaktordruck von 236 bar, einer Reaktortemperatur von 406°C und einem Volumenstrom von 65 ml/min ein Umsatz des 2-Propanols von 96,1%; die Selektivität bezüglich Propen errechnete sich zu 97,8%.

### Beispiel 9:

Analog zu obenstehendem Beispiel 8 wurde der mit demselben Katalysator (γ-Aluminiumoxid) Reaktor 7 mit einer wässrigen Eduktlösung von 6,06 Mass.-% 2-Propanol betrieben. Die in dem Reaktor 7 eingestellten Betriebsparameter waren eine Temperatur von 404°C, ein Druck von 241 bar und ein Volumenstrom von 65 ml/min. Hieraus resultierte ein Umsatz von 98,57% bei einer Selektivität zu Propen von 98,01%.

### Beispiel 10:

Es wurden dem Reaktor 7 59,4 g γ-Aluminiumoxid als heterogenem Dehydratisierungskatalysator aufgegeben Hieraus ergab sich ein freies Reaktorvolumen von 101,0 ml. Der Reaktor wurde mit einer wässrigen Eduktlösung aus 40 Mass.-% 1-Propanol beschickt; die Reaktionstemperatur betrug 408°C und der Reaktionsdruck 251 bar. Der Volumenstrom der Pumpe 3 betrug wiederum 30 ml/min. Durch diese Betriebsweise ergab sich ein Umsatz des 1-Propanols von 53,11%, die Selektivität zu Propen belief sich auf 94,04%.

### Vergleichsbeispiel:

Zum Vergleich wurden Versuche mit leerem Reaktionsrohr und mit herkömmlichen SiC-Materialien befülltem Reaktor 7 durchgeführt. Die Reaktionsbedingungen im Reaktor 7 wurden bei diesen Versuchen auf 250 bar und 400°C eingestellt. Als Eduktstrom wurde eine 40 Mass.-%ige wässrige 2-Propanollösung mit einem Volumenstrom von wiederum 30 ml/min eingesetzt. Hierbei hat keine nennenswerte Reaktion stattgefunden; ein Umsatz war nicht messbar. Während des Versuches hat sich in dem Phasenseparator 14 keine Gasphase ausgebildet, woraufhin der Versuch abgebrochen wurde.

## Patentansprüche

1. Verfahren zur Herstellung von Propen mittels Dehydratisierung von 1-Propanol und/oder 2-Propanol an heterogenen Dehydratisierungskatalysatoren, umfassend die folgenden Schritte:
(a) Bereitstellen eines 1-Propanol und/oder 2-Propanol enthaltenden Eduktes und Erwärmen des Eduktes auf wenigstens 374°C sowie Beaufschlagen des Eduktes mit einem Druck von wenigstens 221 bar;
(b) Überführen des Eduktes in wenigstens einen mit wenigstens einem heterogenen Dehydratisierungskatalysator befüllten Reaktor (7) und Inkontaktbringen des Eduktes mit dem Dehydratisierungskatalysator unter Aufrechterhaltung einer Temperatur von wenigstens 374°C sowie einem Druck von wenigstens 221 bar, so dass ein Propen enthaltendes Produkt erhalten wird;
(c) Entspannen des Produktes auf höchstens 47 bar sowie Abkühlen des Produktes auf höchstens 90°C stromab des Reaktors (7) und Überführen des Produktes in einen Phasenseparator (14); und
(d) Entnehmen des Propens aus der Gasphase des Phasenseparators (14) und Entnehmen zumindest des bei der Dehydratisierungsreaktion gebildeten Wassers aus der Flüssigphase des Phasenseparators (14) unter Aufrechterhaltung einer Temperatur von höchstens 90°C sowie einem Druck von höchstens 47 bar in dem Phasenseparator (14).

2. Verfahren nach Anspruch 1, **dadurch gekennzeichnet, dass** es kontinuierlich durchgeführt wird, wobei es die folgenden Schritte umfasst:
(a) Bereitstellen eines 1-Propanol und/oder 2-Propanol enthaltenden Eduktstromes und Erwärmen des Eduktstromes auf wenigstens 374°C sowie Beaufschlagen des Eduktstromes mit einem Druck von wenigstens 221 bar;
(b) Überführen des Eduktstromes in wenigstens einen mit wenigstens einem heterogenen Dehydratisierungskatalysator befüllten Reaktor (7), insbesondere in Form eines Rohrreaktors, eines Rohrbündelreaktors oder einer Kaskade der vorgenannten Reaktoren, und Inkontaktbringen des Eduktstromes mit dem Dehydratisierungskatalysator unter Aufrechterhaltung einer Temperatur von wenigstens 374°C sowie einem Druck von wenigstens 221 bar, so dass ein Propen enthaltender Produktstrom erhalten wird;
(c) Entspannen des Produktstromes auf höchstens 47 bar sowie Abkühlen des Produktstromes auf höchstens 90°C stromab des Reaktors (7) und Überführen des Produktstromes in einen Phasenseparator (14); und
(d) Entnehmen eines Propenstromes aus der Gasphase des Phasenseparators (14) und Entnehmen zumindest eines Flüssigkeitsstromes, welcher das bei der Dehydratisierungsreaktion gebildete Wasser enthält, aus der Flüssigphase des Phasenseparators (14) unter Aufrecht erhaltung einer Temperatur von höchstens 90°C sowie einem Druck von höchstens 47 bar in dem Phasenseparator (14).

3. Verfahren nach Anspruch 1 oder 2, **dadurch gekennzeichnet, dass** als Edukt eine wässrige Lösung von 1-Propanol und/oder 2-Propanol, insbesondere in einer Konzentration zwischen 5 und 98 Mass.-% 1- und/oder 2-Propanol bezogen auf die Gesamtlösung, eingesetzt wird.

4. Verfahren nach einem der Ansprüche 1 bis 3, **dadurch gekennzeichnet, dass** in dem Reaktor (7) eine Temperatur zwischen 374°C und 500°C und/oder ein Druck zwischen 221 bar und 350 bar eingestellt wird.

5. Verfahren nach einem der Ansprüche 1 bis 4, **dadurch gekennzeichnet, dass** das Produkt stromab des Reaktors (7) auf eine Temperatur zwischen 5°C und 90°C abgekühlt und/oder auf einen Druck zwischen 1 bar und 46 bar entspannt wird.

6. Verfahren nach einem der Ansprüche 1 bis 5, **dadurch gekennzeichnet, dass** das Produkt stromab des Reaktors (7) auf eine Temperatur zwischen 60°C und 90°C abgekühlt und/oder auf einen Druck zwischen 30 bar und 46 bar entspannt wird.

7. Verfahren nach einem der Ansprüche 1 bis 6, **dadurch gekennzeichnet, dass** zumindest ein Teil der dem Phasenseparator (14) entnommenen flüssigen Phase auf wenigstens 374°C erwärmt sowie mit einem Druck von wenigstens 221 bar beaufschlagt und in den Reaktor (7) rezirkuliert wird.

8. Verfahren nach einem der Ansprüche 1 bis 7, **dadurch gekennzeichnet, dass** das dem Phasenseparator (14) entnommene Propen einem Abfüll- und/oder Weiterverarbeitungsprozess zugeführt wird.

9. Verfahren nach einem der Ansprüche 1 bis 8, **dadurch gekennzeichnet, dass** als heterogener Katalysator wenigstens ein Katalysator auf der Basis von Metalloxiden aus der Gruppe der Metalle aus der vierten Nebengruppe und aus der dritten Hauptgruppe des Periodensystems, insbesondere aus der Gruppe Aluminium, Zirconium und Titan, eingesetzt wird.

10. Verfahren nach Anspruch 9, **dadurch gekennzeichnet, dass** als heterogener Katalysator wenigstens ein Katalysator aus der Gruppe Aluminiumoxid (Al₂O₃), insbesondere der Modifikation γ-Aluminiumoxid und/oder θ-Aluminiumoxid, Zirconiumdioxid (ZrO₂), insbesondere in tetragonaler Modifikation, und Titandioxid (TiO₂), insbesondere in tetragonale holoedrischer Modifikation (Anatas), eingesetzt wird.

11. Verfahren nach einem der Ansprüche 1 bis 10, **dadurch gekennzeichnet, dass** wenigstens ein heterogener Katalysator mit einer spezifischen Oberfläche zwischen 10 m²/g und 400 m²/g, ermittelt nach BET (Brunauer, Emmett, Teller), eingesetzt wird.

12. Verfahren nach einem der Ansprüche 1 bis 11, **dadurch gekennzeichnet, dass** der Reaktor (7) mit einer Beladung des heterogenen Katalysators zwischen 0,001 und 3 g_{Propanol}/g_{Katalysator} · min, insbesondere zwischen 0,005 und 2 g_{Propanol}/g_{Katalysator} · min, versehen wird.

13. Verfahren nach einem der Ansprüche 1 bis 12, **dadurch gekennzeichnet, dass** das eingesetzte 1-Propanol und/oder 2-Propanol aus nachwachsenden Rohstoffen, insbesondere mittels Fermentation, gewonnen wird.

## Claims

1. Process for preparing propene by dehydration of 1-propanol and/or 2-propanol over heterogeneous dehydration catalysts, which comprises the following steps:
(a) providing a starting material containing 1-propanol and/or 2-propanol and heating the starting material to at least 374°C and applying a pressure of at least 221 bar to the starting material;
(b) transferring the starting material into at least one reactor (7) charged with at least one heterogeneous dehydration catalyst and contacting the starting material with the dehydration catalyst while maintaining a temperature of at least 374°C and a pressure of at least 221 bar so as to give a propene-containing product;
(c) depressurizing the product to not more than 47 bar and cooling the product to not more than 90°C downstream of the reactor (7) and transferring the product into a phase separator (14); and
(d) taking off the propene from the gas phase of the phase separator (14) and taking off at least the water formed in the dehydration reaction from the liquid phase of the phase separator (14) while maintaining a temperature of not more than 90°C and a pressure of not more than 47 bar in the phase separator (14).

2. Process according to Claim 1, **characterized in that** it is carried out continuously and comprises the following steps:
(a) providing a starting material stream containing 1-propanol and/or 2-propanol and heating the starting material to at least 374°C and applying a pressure of at least 221 bar to the starting material stream;
(b) transferring the starting material stream into at least one reactor (7) charged with at least one heterogeneous dehydration catalyst, in particular in the form of a tube reactor, a shell-and-tube reactor or a cascade of the abovementioned reactors, and contacting the starting material stream with the dehydration catalyst while maintaining a temperature of at least 374°C and a pressure of at least 221 bar so as to give a propene-containing product stream;
(c) depressurizing the product stream to not more than 47 bar and cooling the product stream to not more than 90°C downstream of the reactor (7) and transferring the product stream into a phase separator (14); and
(d) taking off the propene stream from the gas phase of the phase separator (14) and taking off at least one fluid stream which contains the water formed in the dehydration reaction from the liquid phase of the phase separator (14) while maintaining a temperature of not more than 90°C and a pressure of not more than 47 bar in the phase separator (14).

3. Process according to Claim 1 or 2, **characterized in that** an aqueous solution of 1-propanol and/or 2-propanol, in particular in a concentration in the range from 5 to 98% by mass of 1- and/or 2-propanol based on the total solution, is used as starting material.

4. Process according to any of Claims 1 to 3, **characterized in that** a temperature in the range from 374°C to 500°C and/or a pressure in the range from 221 bar and 350 bar is set in the reactor (7).

5. Process according to any of Claims 1 to 4, **characterized in that** the product is cooled to a temperature in the range from 5°C to 90°C and/or depressurized to a pressure in the range from 1 bar to 46 bar downstream of the reactor (7).

6. Process according to any of Claims 1 to 5, **characterized in that** the product is cooled to a temperature in the range from 60°C to 90°C and/or depressurized to a pressure in the range from 30 bar to 46 bar downstream of the reactor (7).

7. Process according to any of Claims 1 to 6, **characterized in that** at least part of the liquid phase taken off from the phase separator (14) is heated to at least 374°C and brought to a pressure of at least 221 bar and recirculated to the reactor (7).

8. Process according to any of Claims 1 to 7, **characterized in that** the propene taken off from the phase separator (14) is fed to a packaging and/or further processing process.

9. Process according to any of Claims 1 to 8, **characterized in that** at least one catalyst based on metal oxides from the group of metals of the fourth transition group and from the third main group of the Periodic Table, in particular from the group consisting of aluminium, zirconium and titanium, is used as heterogeneous catalyst.

10. Process according to Claim 9, **characterized in that** at least one catalyst from the group consisting of aluminium oxide (Al₂O₃), in particular in the modification group γ-aluminium oxide and/or θ-aluminium oxide, zirconium dioxide (ZrO₂), in particular in the tetragonal modification, and titanium dioxide (TiO₂), in particular in the tetragonal holohedral modification (anatase), is used as heterogeneous catalyst.

11. Process according to any of Claims 1 to 10, **characterized in that** at least one heterogeneous catalyst having a specific surface area in the range from 10 m²/g to 400 m²/g, determined by the BET (Brunauer, Emmett, Teller) method, is used.

12. Process according to any of Claims 1 to 11, **characterized in that** the reactor (7) is provided with a loading of the heterogeneous catalyst in the range from 0.001 to 3 gₚᵣₒₚₐₙₒₗ/g_{catalyst} ·min, in particular in the range from 0.005 to 2 gₚᵣₒₚₐₙₒₗ/g_{catalyst} ·min.

13. Process according to any of Claims 1 to 12, **characterized in that** the 1-propanol and/or 2-propanol used is obtained from renewable raw materials, in particular by means of fermentation.

## Revendications

1. Procédé de fabrication de propène par déshydratation de 1-propanol et/ou 2-propanol au niveau de catalyseurs de déshydratation hétérogènes, comprenant les étapes suivantes :
(a) mise à disposition d'un produit de départ contenant du 1-propanol et/ou 2-propanol et chauffage du produit de départ à au moins 374 °C ainsi que mise sous pression du produit de départ à une pression d'au moins 221 bar ;
(b) transfert du produit de départ dans au moins un réacteur (7) rempli d'au moins un catalyseur de déshydratation hétérogène et mise en contact du produit de départ avec le catalyseur de déshydratation en maintenant une température d'au moins 374 °C ainsi qu'une pression d'au moins 221 bar, de façon à obtenir un produit contenant du propène ;
(c) détente du produit à tout au plus 47 bar ainsi que refroidissement du produit à tout au plus 90 °C en aval du réacteur (7) et transfert du produit dans un séparateur de phases (14) ; et
(d) retrait du propène hors de la phase gazeuse du séparateur de phases (14) et retrait au moins de l'eau formée lors de la réaction de déshydratation hors de la phase fluide du séparateur de phases (14) en maintenant une température de tout au plus 90 °C ainsi qu'une pression de tout au plus 47 bar dans le séparateur de phases (14).

2. Procédé selon la revendication 1, **caractérisé en ce que** qu'il est mis en oeuvre en continu et comprend les étapes suivantes :
(a) mise à disposition d'un produit de départ contenant du 1-propanol et/ou du 2-propanol et chauffage du produit de départ à au moins 374 °C ainsi que mise sous pression du produit de départ à une pression d'au moins 221 bar ;
(b) transfert du produit de départ dans au moins un réacteur (7) rempli d'au moins un catalyseur de déshydratation hétérogène, notamment sous la forme d'un réacteur à tube, d'un réacteur à bloc de tubes ou d'une cascade desdits réacteurs et mise en contact du produit de départ avec le catalyseur de déshydratation en maintenant une température d'au moins 374 °C ainsi qu'une pression d'au moins 221 bar, de façon à obtenir un produit//flux contenant du propène ;
(c) détente du flux de produit à tout au plus 47 bar ainsi que refroidissement du flux de produit à tout au plus 90 °C en aval du réacteur (7) et transfert du flux de produit dans un séparateur de phases (14) ; et
(d) retrait d'un flux de produit de propène hors de la phase gazeuse du séparateur de phases (14) et retrait d'au moins un flux fluide contenant l'eau formée lors de la réaction de déshydratation, hors de la phase fluide du séparateur de phases (14) en maintenant une température de tout au plus 90 °C ainsi qu'une pression de tout au plus 47 bar dans le séparateur de phases (14).

3. Procédé selon la revendication 1 ou 2, **caractérisé en ce que** le produit de départ utilisé est une solution aqueuse de 1-propanol et/ou 2-propanol, notamment dans une concentration comprise entre 5 et 98 % en masse de 1-propanol et/ou 2-propanol par rapport à la solution totale.

4. Procédé selon l'une quelconque des revendications 1 à 3, **caractérisé en ce qu'**une température comprise entre 374 °C et 500 °C et/ou une pression comprise entre 221 bar et 350 bar est réglée dans le réacteur (7).

5. Procédé selon l'une quelconque des revendications 1 à 4, **caractérisé en ce que** le produit en aval du réacteur (7) est refroidi à une température comprise entre 5 °C et 90 °C et/ou détendu à une pression comprise entre 1 bar et 46 bar.

6. Procédé selon l'une quelconque des revendications 1 à 5, **caractérisé en ce que** le produit en aval du réacteur (7) est refroidi à une température comprise entre 60 °C et 90 °C et/ou détendu à une pression comprise entre 30 bar et 46 bar.

7. Procédé selon l'une quelconque des revendications 1 à 6, **caractérisé en ce qu'**au moins une partie de la phase fluide prise dans le séparateur de phases (14) est chauffée à au moins 374 °C ainsi que mise sous pression à une pression d'au moins 221 bar et remise en circulation dans le réacteur (7).

8. Procédé selon l'une quelconque des revendications 1 à 7, **caractérisé en ce que** le propène pris dans le séparateur de phases (14) est conduit à une étape de mise en bouteilles et/ou de post-traitement.

9. Procédé selon l'une quelconque des revendications 1 à 8, **caractérisé en ce que** le catalyseur hétérogène utilisé est au moins un catalyseur à base d'oxydes métalliques appartenant au groupe des métaux du quatrième groupe secondaire et du troisième groupe principal du tableau périodique, notamment au groupe contenant l'aluminium, le zirconium et le titane. θ

10. Procédé selon la revendication 9, **caractérisé en ce que** le catalyseur hétérogène utilisé est au moins un catalyseur appartenant au groupe contenant l'oxyde
d'aluminium (Al₂0₃), notamment la modification d'oxyde d'aluminium y et/ou d'oxyde d'aluminium θ, de dioxyde de zirconium (Zr0₂), notamment la modification tétragonale et le dioxyde de titane (Ti0₂), notamment la modification holoédrique tétragonale (Anatas).

11. Procédé selon l'une quelconque des revendications 1 à 10, **caractérisé en ce qu'**au moins un catalyseur hétérogène est utilisé avec une surface spécifique comprise entre 10 m²/g et 400 m²/g, calculée selon BET (Brunauer, Emmett, Teller).

12. Procédé selon l'une quelconque des revendications 1 à 11, **caractérisé en ce que** le réacteur (7) est prévu avec un chargement du catalyseur hétérogène compris entre 0,001 et 3 gₚᵣₒₚₐₙₒₗ/g_{catalyseur} • min, notamment entre 0,005 et 2 gₚᵣₒₚₐₙₒₗ/g_{catalyseur} • min.

13. Procédé selon l'une quelconque des revendications 1 à 12, **caractérisé en ce que** le 1-propanol et/ou 2-propanol utilisé est obtenu à partir de matière première repoussée, notamment par fermentation.
